(19) Europäisches Patentamt
European Patent Office
Office européen des brevets

(11) Publication number: 0 080 314 A1

(12) # EUROPEAN PATENT APPLICATION

(21) Application number: 82306082.7

(22) Date of filing: 16.11.82

(51) Int. Cl.³: C 07 C 77/02, C 07 C 76/04, C 07 H 15/08, C 10 L 1/22, C 08 G 65/32

(30) Priority: 17.11.81 ZA 817961

(43) Date of publication of application: 01.06.83 Bulletin 83/22

(84) Designated Contracting States: DE FR GB SE

(71) Applicant: **AECI LIMITED, 16th Floor Office Tower Carlton Centre Commissioner Street, Johannesburg Transvaal (ZA)**

(72) Inventor: **Stiff, Anthony John, 19 Lois Road, Illiondale Edenvale Transvaal Province (ZA)**

(74) Representative: **Martin, David Lincoln et al, Imperial Chemical Industries PLC Legal Department: Patents Thames House North Millbank, London SW1P 4QG (GB)**

(54) **Nitrato-polyethers, and their use.**

(57) The invention concerns an organic nitrato compound of the general formula (I)

$$X_m(R_f)[(OA)_n\ X]_{f-m} \qquad (I)$$

in which

$R_f$ is an organic residue containing 3 or more carbon atoms derived from a base polyhydroxy compound of formula $R(OH)_f$ by removal of OH, where f denotes the number of hydroxyl functions on the molecule;

One or more of X are nitrate groups ($-ONO_2$), the remainder being hydroxyl ($-OH$);

n is an integer between 1 and 100, and may vary in a particular compound where f–m is greater than 1;

OA is an oxyalkylene unit derived from ethylene oxide or propylene oxide;

m is an integer in the range 0 to f–1;

f is an integer in the range 2 to 10, and is always equal to or less than the number of carbon atoms in the radical R.

The compound can be used as additives to fuels, alcohols, combustible fluids or the like.

EP 0 080 314 A1

Z/H.32511/EP.

THIS INVENTION relates to novel organic nitrato compounds, processes for their preparation, and their use.

The present invention provides an organic nitrato compound of the general formula (I)

$$X_m \, (R_f) \, \left[(OA)_n \, X\right]_{f-m} \qquad (I)$$

in which

$R_f$ is an organic residue containing 3 or more carbon atoms derived from a base polyhydroxy compound of formula $R(OH)_f$ by removal of OH, where f denotes the number of hydroxyl functions on the molecule;

One or more of X are nitrate groups ($-ONO_2$), the remainder being hydroxyl (-OH);

n is an integer between 1 and 100, and may vary in a particular compound where f-m is greater than 1;

OA is an oxyalkylene unit derived from ethylene oxide or propylene oxide;

m is an integer in the range 0 to f-1;

f is an integer in the range 2 to 10, and is always equal to or less than the number of carbon atoms in the radical R.

The compounds of the above general formula (I) are nitric acid esters of alkylene and polyalkylene glycol ethers of polyols.

The novel compounds provided by the invention may for example be used as ignition improvers for compression-ignition fuels, as anti-corrosion agents, as anti-knock agents for spark-ignition fuels, as components of fuel, propellant and explosive compositions, and the like. They are particularly useful as ignition improvers for enabling non-petroleum-based fuels to be used for compression ignition engines.

The invention also provides a process for the preparation of the organic nitrato compounds of the general formula (I) above. The organic nitrato compounds can be prepared by reacting a polyhydroxy compound of general formula (I), (in which at least some of the groups X are hydroxyl the remainder, if any, being nitrate groups) with nitric acid, preferably under dehydrating conditions.

The polyhydroxy compounds of formula (I) in which all X are hydroxyl, may be prepared by reaction of ethylene oxide or propylene oxide with a base polyhydroxy compound of formula $R(OH)_f$.

The reactions of ethylene oxide and propylene oxide with hydroxy compounds generally requires elevated temperatures, pressures above atmospheric, and the presence of catalysts.

Selective addition of the alkylene oxide to the base polyhydroxy compound $R(OH)_f$ may be achieved by adjustment of reaction conditions or by chemically blocking one or more hydroxyl groups.

Under mild reaction conditions, the alkylene oxide units can be added only to the most reactive hydroxyl groups, thus forming a molecule with alkylene oxide units or chains attached to these groups, the less reactive hydroxyl groups remaining unreacted.

By adjusting reaction stoichiometry such that there are less than f alkylene oxide molecules per base polyhydroxy molecule, addition of single alkylene oxide units to the most reactive hydroxyl groups can be achieved.

By chemically blocking the most reactive hydroxyl groups on the base polyhydroxy molecule, for example using ester groups, the alkylene oxide can be added to the less reactive (ie unblocked) hydroxyl groups. The blocked hydroxyls can then be regenerated, for example by hydrolysis of the ester groups.

Other methods of preparation of the compounds of formula I, in which all X are hydroxyl, include the reaction of ethylene glycol or 'blocked' ethylene glycols (such as chloroethanol, ethylene glycol monoacetate and 2-tetrahydropyranoxyethanol) with epoxides, followed by removal of blocking groups, if any.

Nitration can then be effected to provide the compounds of the invention, of general formula (I).

The nitration with nitric acid conveniently may be carried out under dehydrating conditions, eg in the presence of sulphuric acid or acetic anhydride, preferably at temperatures where minimal oxidation takes place, eg at temperatures from about $-10^{o}C$ to about $+30^{o}C$. Generally, a mixture of compounds of general formula (I) is formed. When carrying out the nitration, the excess acidity may subsequently be neutralised by means of an alkali.

The organic nitrato compounds of general formula (I) contain one or more nitrato groups, but preferably more than 90% of the hydroxyl groups are nitrated. The integer n varies from 1 to 100 and more usually is from 1 to 10, particularly from 1 to 5, and may vary in a molecule where f-m is greater than 1.

In the above general formula, R is an organic radical derived from a base polyhydroxy compound of formula $R(OH)_f$, containing three or more carbon atoms, and may optionally also contain non-hydroxyl oxygen.

Examples of base polyhydroxy compounds $R(OH)_f$ are:

(a) Sugars, such as
- (i) the tetroses, for example erithrose,
- (ii) the pentoses, for example xylose,
- (iii) the hexoses, for example sorbose, etc.,
- (iv) disaccharides, for example sucrose.

(b) Polyhydroxy compounds which contain one hydroxyl group per carbon atom, such as
- (i) glycerol,
- (ii) the tetritols, eg erithritol,
- (iii) the pentitols, eg xylitol,
- (iv) the hexitols, eg sorbitol, etc.

(c) Polyhydroxy alkanes which contain less than one hydroxyl group per carbon atom, such as
- (i) trihydroxyalkanes containing 4 or more C-atoms, eg trimethylol ethane, trimethylol propane, 1,2,5 trihydroxy pentane,
- (ii) tetrahydroxyalkanes containing 5 or more C-atoms, eg pentaerithritol.

(d) Polyhydroxy compounds containing ether linkages, eg

(i) the polyglycerols

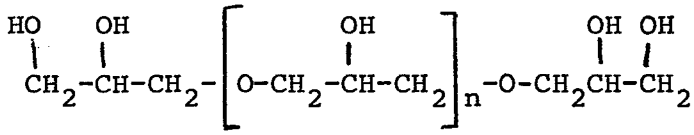

(ii) dipentaerithritol

$(HOCH_2)_3C-CH_2OCH_2-C(CH_2OH)_3$

(iii) compounds of the type

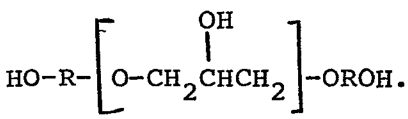

(e) Diols such as propane-1,3-diol, butane-1,4-diol, butane-2,3-diol, cyclohexane-1,4-diol, etc.

Some Examples of compounds of the invention of Formula (I) are:

1. $O_2NO(CH_2CH_2O)_x - (C_yH_{2y}) - (OCH_2CH_2)_z-ONO_2$.

where $x = 1$ to $50$

$y = 3$ to $20$

$z = 0$ to $50$

eg 1.1 $O_2NO-CH_2CH_2O-CH_2CH_2CH_2ONO_2$

1.2 $O_2NO-CH_2CH_2O-CH_2CH_2CH_2-O-CH_2CH_2-ONO_2$

1.3 $O_2NO-(CH_2CH_2O)_2-CH_2CH_2CH_2-CH_2-O-CH_2CH_2-ONO_2$

1.4 $O_2NO-CH_2CH_2O-CH(CH_3)-CH(CH_3)-OCH_2CH_2\ ONO_2$, or

1.5 $O_2NO-CH_2CH_2O-(CH_2)_5-(O-CH_2CH_2)_2\ ONO_2$.

2. 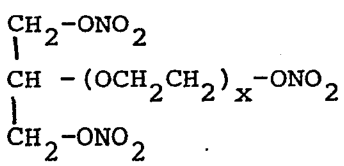

where x = 1 to 100

eg 2.1) $(O_2NOCH_2)_2CH\text{-}OCH_2CH_2ONO_2$ or

2.2) $(O_2NOCH_2)_2CH\text{-}(OCH_2CH_2)_3ONO_2$.

3)
$$\begin{array}{l} CH_2 - (OCH_2CH_2)_x\text{-}ONO_2 \\ | \\ CH - (OCH_2CH_2)_y\text{-}ONO_2 \\ | \\ CH_2 - (OCH_2CH_2)_z\text{-}ONO_2 \end{array}$$

where x = 1 to 30

y = 0 to 30

z = 0 to 30

eg 3.1)
$$\begin{array}{l} CH_2\text{-}(OCH_2CH_2)ONO_2 \\ | \\ CH - ONO_2 \\ | \\ CH_2 - ONO_2 \end{array}$$

4) A compound of the formula

$$\begin{array}{l} \quad\;\; CH_2 - (OCH_2CH_2)_x\text{-}ONO_2 \\ \quad\;\; | \\ R - C \;\; - (OCH_2CH_2)_y\text{-}ONO_2 \\ \quad\;\; | \\ \quad\;\; CH_2 - (OCH_2CH_2)_z\text{-}ONO_2 \end{array}$$

where x is 1 to 20

y is 0 to 20

z is 0 to 20

and R is hydrogen or $C_1$ to $C_4$ alkyl.

5.
$$\begin{array}{l} CH_2 - (OCH_2CH_2)_x\text{-}ONO_2 \\ | \\ CH - ONO_2 \\ | \\ CH - ONO_2 \\ | \\ CH_2 - (OCH_2CH_2)_y\text{-}ONO_2 \end{array}$$

where $x = 1$ to 50

$y = 0$ to 50.

6) A compound of the formula

$$CH_2-(OCH_2CH_2)_w-ONO_2$$
$$|$$
$$CH-(OCH_2CH_2)_x-ONO_2$$
$$|$$
$$CH-(OCH_2CH_2)_y-ONO_2$$
$$|$$
$$CH_2-(OCH_2CH_2)_z-ONO_2$$

where w is 0 to 20

x is 0 t0 20

y is 0 to 20

z is 0 to 20

provided $w+x+y+z \geqslant 1$.

7).

$$CH_2ONO_2$$
$$|$$
$$CH-(OCH_2CH_2)_x-ONO_2$$
$$|$$
$$CH-(OCH_2CH_2)_y-ONO_2$$
$$|$$
$$CH_2-ONO_2$$

where $x = 1$ to 50

$y = 0$ to 50

eg

$$CH_2-ONO_2$$
$$|$$
$$CH-(OCH_2CH_2)-ONO_2$$
$$|$$
$$CH-(OCH_2CH_2)-ONO_2$$
$$|$$
$$CH_2-ONO_2.$$

8) Compounds similar to Examples 5, 6 and 7, but based on pentitols and hexitols.

9)

$$CH_2-(OCH_2CH_2)_v-ONO_2$$
$$|$$
$$CH-(OCH_2CH_2)_w-ONO_2$$
$$|$$
$$CH-(OCH_2CH_2)_x-ONO_2$$
$$|$$
$$CH-(OCH_2CH_2)_y-ONO_2$$
$$|$$
$$CH_2-(OCH_2CH_2)_z-ONO_2$$

where $v = 0$ to 20
$w = 0$ to 20
$x = 0$ to 20
$y = 0$ to 20
$z = 0$ to 20
provided $v+w+x+y+z \geqslant 1$.

10) A compound comprising the nitric acid ester of an adduct of a pentose monosaccharide with ethylene oxide, containing between 1 and 50 oxyethylene groups.

11) A compound comprising the nitric acid ester of an adduct of the hexose monosaccharide with ethylene oxide, containing between 1 and 50 oxyethylene groups.

12). Pentaerithritol-based compounds:

$$CH_2-(OCH_2CH_2)_w-ONO_2$$
$$|$$
$$O_2NO(CH_2CH_2O)_zCH_2-C-CH_2(OCH_2CH_2)_x-ONO_2$$
$$|$$
$$CH_2-(OCH_2CH_2)_y-ONO_2$$

where $w = 1$ to 25
$x = 0$ to 25
$y = 0$ to 25
$z = 0$ to 25.

The invention also provides compositions containing an organic nitrato compound of formula (I) and another substance compatible therewith. Such compositions may contain, as the other substance, a fuel, whether of

mineral origin or vegetable origin (eg diesel fuels, petroleum distillates, a product obtained from the conversion of coal to coal-liquids, vegetable oils, and the like), an alcohol (eg methanol, ethanol and the like), a combustible fluid or another suitable organic or inorganic material. The compositions may conveniently contain from about 0,01 to 50% by weight of a compound of general formula (I). Some of these compositions may be used as fuels.

The invention also provides a fuel for compression ignition engines, said fuel comprising from 0,01 to 10% by weight of a compound of formula (I) in admixture with a combustible fluid.

The combustible fluid present in the fuel may be one or more of methanol, ethanol, a $C_2$ to $C_{10}$ alcohol or a low cetane hydrocarbon, ie a hydrocarbon which, in the absence of a compound of the invention, cannot be used as a fuel in a compression-ognition engine, but when mixed with a compound of the invention, can be used as such a fuel.

As indicated above, the compounds of general formula (I) may be a mixture. Similarly, any material admixed therewith may also be a mixture. When an alkanol is present, there may be one or more alkanols having from 1 to 20 carbon atoms each.

The invention also provides a method of improving the combustion of fuels, propellants or explosives, which comprises admixing therewith 0,01 to 50% by weight of a nitrato compound of general formula (I), and combusting and/or detonating the resultant mixture.

The invention further provides a method of running an engine, which comprises injecting into the engine, either separately or in combination, a nitrato

compound of the general formula (I) and a combustible fuel. Conveniently, the combustible fuel and the organic nitrato compound may be injected into the engine together.

The invention is illustrated in non-limiting manner by reference to the following Examples:

EXAMPLE 1

0,2 moles of a mixture of the two isomeric compounds obtained from the addition of two moles of ethylene oxide to one mole of glycerol (ie 4,7-dioxanonane 1,2,9 triol and 3,7-dioxanonone 1,5,9 triol) was added to 120 ml of 28% $HNO_3$ in acetic anhydride at $0^oC$ over one hour. The reaction mixture was quenched in 300 ml water, the organic layer separated, neutralised with a dilute aqueous ammonia, washed with water, and dried to yield a fully nitrated product A in 85% yield. Product A comprises a mixture of Al and A2:

Al $CH_2-OCH_2CH_2ONO_2$
|
$CH -ONO_2$
|
$CH_2-OCH_2CH_2-ONO_2$

A2 $CH_2-O-CH_2CH_2-OCH_2CH_2ONO_2$
|
$CH -ONO_2$
|
$CH_2ONO_2$.

The infra-red spectrum and liquid chromatography scans of the product were consistent with that expected of a mixture of Al and A2.

EXAMPLE 2

A compound identical to one obtained from the addition of one mole of ethylene oxide to one mole of glycerol (ie 4-oxahexane 1,2,6 triol) was obtained by slowly adding 1

mole of epichlorohydrin to 3 moles of ethylene glycol containing 0,2% $BF_3$ etherate at 90°C, and hydrolysing the resultant adduct with 5% aqueous sodium hydroxide at reflux. Water and excess ethylene glycol were removed from the neutralised mixture by distillation, and inorganic salts by filtration. 0,2 moles of the product was nitrated by the method of Example 1 to give a product B in 84% yield.

B is
$CH_2-OCH_2CH_2ONO_2$
|
$CH\ -ONO_2$
|
$CH_2-ONO_2$.

The IR spectrum and LC scan of product B were consistent with this structure.

EXAMPLE 3

Fuels comprising 3,5% of the test compound, 1% castor oil and 95,5% methanol were used to operate an instrumented single-cylinder air-cooled direct-injection diesel engine of 15,7:1 compression ratio driving a 230V 15A generator. The test compounds were i) product A from Example 1; and ii) product B from Examples 2. A conventional 45-cetane hydrocarbon diesel fuel was used for comparison.

In all cases the engine started easily from cold, idled smoothly, and ran normally under all load conditions. The display of cylinder pressure indicated that the combustion characteristics were satisfactory for normal operation of the engine.

EXAMPLE 4

A mixture of compounds obtained from the addition of 4 moles of ethylene oxide to 1 mole of glycerol was nitrated by the method of Example 1, using 1,5 moles $HNO_3$ and 2,0 moles acetic anhydride per equivalent of hydroxyl, to form a mixture of products C1 of formula C:-

$$\begin{array}{l} CH_2-(OCH_2CH_2)_x-ONO_2 \\ | \\ CH-\ (OCH_2CH_2)_y-ONO_2 \\ | \\ CH_2-(OCH_2CH_2)_z-ONO_2 \end{array} \qquad (C)$$

in which the mean value of x+y+z is 4.

EXAMPLE 5

The procedure of Example 4 was repeated using a mixture of compounds obtained from the addition of 6 moles of ethylene oxide to 1 mole of glycerol, to form a mixture of products $C_2$, of formula C in which the mean value of x+y+z is 6.

EXAMPLE 6

Two compounds, obtained from the addition of 4 and 6 moles respectively, of ethylene oxide to 1 mole of pentaerithritol, was nitrated according to the method of Example 4, to yield products D1 and D2 respectively, of formula

$$\begin{array}{c} CH_2-(OCH_2CH_2)_x-ONO_2 \\ | \\ O_2NO-(CH_2CH_2O)_w-CH_2-C-CH_2-(OCH_2CH_2)_y-ONO_2 \\ | \\ CH_2-(OCH_2CH_2)_z-ONO_2 \end{array}$$

The mean value of (w+x+y+z) was 4 for product D1 and 6 for product D2.

EXAMPLE 7

A compound, obtained from the addition of 6 moles of ethylene oxide to 1 mole of xylitol, was nitrated by the method of Example 4 to yield a product E of formula

$$\begin{array}{l} CH_2-(OCH_2CH_2)_v-ONO_2 \\ | \\ CH-(OCH_2CH_2)_w-ONO_2 \\ | \\ CH-(OCH_2CH_2)_x-ONO_2 \\ | \\ CH-(OCH_2CH_2)_y-ONO_2 \\ | \\ CH_2-(OCH_2CH_2)_z-ONO_2 \end{array} \qquad (E)$$

in which the mean value of (v+w+x+y+z) was 6.

EXAMPLE 8

The ability of the products of Examples 4, 5, 6 and 7 that is products C1, C2, D1, D2 and E, to improve the compression ignition characteristics of fuels was demonstrated by preparing fuels comprising 3,5% by volume of product, 1% by volume castor oil, (as lubricant) and 95,5% methanol. Whereas methanol, alone or with castor oil, is a very poor compression-ignition fuel, and will not operate a compression-ignition engine, the five prepared fuels containing products C1, C2, D1, D2 and E operated the engine of Example 3 in a satisfactory manner. Using these prepared fuels, the engine started easily from cold, idled smoothly, and ran normally under all load conditions. The variation of cylinder pressure with crank angle demonstrated that the ignition and combustion characteristics were similar to that obtained when the engine was operated on normal hydrocarbon diesel fuel.

EXAMPLE 9

A matrix of 28 fuels was prepared of composition:-

3,5% ignition improving material of the invention

1% castor oil (for lubrication purposes)

95,5% combustible fluid which, alone or with castor oil, will not operate a compression-ignition engine.

The ignition improving materials were:-

a) Product A of Example 1
b) Product B of Example 2
c) Product C1 of Example 4
d) Product C2 of Example 5
e) Product D1 of Example 6
f) product D2 of Example 6
g) Product E of Example 7

The combustible fluids were

i) Ethanol
ii) 40% methanol, 40% ethanol, 20% $C_3$ and higher alcohols
iii) 50% methanol, 50% gasoline of boiling range 80-180$^{o}$C
iv) Gasoline of boiling range 80-180$^{o}$C

In all cases, the fuel compositions operated the compression-ignition engine of Example 3 in a satisfactory manner, to give performance similar to that obtained with 45 cetane hydrocarbon diesel fuel.

EXAMPLE 10

The products A,B,C1,C2,D1,D2 and E were obtained from the respective starting materials specified in Examples 1, 2, 4, 5, 6 and 7 by reacting these hydroxylic starting materials, with nitric acid in the presence of sulphuric acid. In all cases, 2 moles of 98% $HNO_3$ and 0,75 moles 98% $HNO_3$ were used per mole of hydroxyl, on a 0,2 mole scale. A nitrating acid was prepared by mixing the nitric and sulphuric acids, and the hydroxylic starting material added to this whilst maintaining the temperature at 0-10$^{o}$C. The reacted mixture was dumped into three times it's volume of iced water, and the product separated and neutralised. Products were shown to be the same as those obtained from nitric acid/acetic anhydride nitration by infra-red spectroscopy and liquid chromatography.

CLAIMS

1. An organic nitrato compound of the general formula (I)

$$X_m\ (R_f)\ \left[(OA)_n\ X\right]_{f-m} \qquad (I)$$

in which

$R_f$ is an organic residue containing 3 or more carbon atoms derived from a base polyhydroxy compound of formula $R(OH)_f$ by removal of OH, where f denotes the number of hydroxyl functions on the molecule;

One or more of X are nitrate groups ($-ONO_2$), the remainder being hydroxyl (-OH);

n is an integer between 1 and 100, and may vary in a particular compound where f-m is greater than 1;

OA is an oxyalkylene unit derived from ethylene oxide or propylene oxide;

m is an integer in the range 0 to f-1;

f is an integer in the range 2 to 10, and is always equal to or less than the number of carbon atoms in the radical R.

2. A compound of the formula

$$O_2NO(CH_2CH_2O)_x-(C_yH_{2y})-(OCH_2CH_2)_zONO_2$$

where x = 1 to 50
y = 3 to 20
z = 0 to 50.

3. A compound as claimed in Claim 2, said compound being

$O_2NO-CH_2CH_2O-CH_2CH_2CH_2ONO_2$

$O_2NO-CH_2CH_2O-CH_2CH_2CH_2-O-CH_2CH_2-ONO_2$

$O_2NO-(CH_2CH_2O)_2-CH_2CH_2CH_2-CH_2-O-CH_2CH_2-ONO_2$

$O_2NO-CH_2CH_2O-CH(CH_3)-CH(CH_3)-OCH_2CH_2\ ONO_2$, or

$O_2NO-CH_2CH_2O-(CH_2)_5-(O-CH_2CH_2)_2\ ONO_2$.

4. A compound of the formula

$CH_2-ONO_2$
$CH-(OCH_2CH_2)_x-ONO_2$ where x = 1 to 100
$CH_2-ONO_2$.

5. A compound as claimed in Claim 4, wherein the compound is

$(O_2NOCH_2)_2CH-OCH_2CH_2ONO_2$ or
$(O_2NOCH_2)_2CH-(OCH_2CH_2)_3ONO_2$.

6. A compound of the formula

$CH_2 - (OCH_2CH_2)_x-ONO_2$
$CH - (OCH_2CH_2)_y-ONO_2$
$CH_2 - (OCH_2CH_2)_z-ONO_2$

where x = 1 to 30
y = 0 to 30
z = 0 to 30.

7. The compound

$CH_2-(OCH_2CH_2)ONO_2$
$CH - ONO_2$
$CH_2 - ONO_2$.

8. A compound of the formula

$CH_2 -(OCH_2CH_2)_x- ONO_2$
$R-C$ — $(OCH_2CH_2)_y- ONO_2$
$CH_2 -(OCH_2CH_2)_z- ONO_2$

where x is 1 to 20
y is 0 to 20
z is 0 to 20
and R is hydrogen on $C_1$ to $C_4$ alkyl.

9. A compound of the formula

$$CH_2 - (OCH_2CH_2)_x - ONO_2$$
$$|$$
$$CH - ONO_2$$
$$|$$
$$CH - ONO_2$$
$$|$$
$$CH_2 - (OCH_2CH_2)_y - ONO_2$$

where $x = 1$ to $50$

$y = 0$ to $50$.

10. A compound of the formula

$$CH_2 - (OCH_2CH_2)_w - ONO_2$$
$$|$$
$$CH - (OCH_2CH_2)_x - ONO_2$$
$$|$$
$$CH - (OCH_2CH_2)_y - ONO_2$$
$$|$$
$$CH_2 - (OCH_2CH_2)_z - ONO_2$$

where w is o to 20

x is 0 to 20

y is 0 to 20

z is 0 to 20

provided $w+x+y+z \geq 1$

11. A compound of the formula

$$CH_2ONO_2$$
$$|$$
$$CH - (OCH_2CH_2)_x - ONO_2$$
$$|$$
$$CH - (OCH_2CH_2)_y - ONO_2$$
$$|$$
$$CH_2 - ONO_2$$

where $x = 1$ to $50$

$y = 0$ to $50$.

12. The compound

$CH_2-ONO_2$
$CH-(OCH_2CH_2)-ONO_2$
$CH-(OCH_2CH_2)-ONO_2$
$CH_2-ONO_2$.

13. A compound of the formula

$CH_2-(OCH_2CH_2)_v-ONO_2$
$CH-(OCH_2CH_2)_w-ONO_2$
$CH-(OCH_2CH_2)_x-ONO_2$
$CH-(OCH_2CH_2)_y-ONO_2$
$CH-(OCH_2CH_2)_z-ONO_2$

where $v = 0$ to 20
$w = 0$ to 20
$x = 0$ to 20
$y = 0$ to 20
$z = 0$ to 20
provided $v+w+x+y+z \geqslant 1$.

14. A compound comprising the nitric acid ester of an adduct of a pentose monosaccharide with ethylene oxide, containing between 1 and 50 oxyethylene groups.

15. A compound comprising the nitric acid ester of an adduct of a hexose monosaccharide with ethylene oxide, containing between 1 and 50 oxyethylene groups.

16. A compound of the formula

$CH_2-(OCH_2CH_2)_w-ONO_2$
$O_2NO(CH_2CH_2O)_zCH_2-C-CH_2(OCH_2CH_2)_x-ONO_2$
$CH_2-(OCH_2CH_2)_y-ONO_2$

where $w = 1$ to 25
$x = 0$ to 25
$y = 0$ to 25
$z = 0$ to 25

17. A composition comprising a compound as claimed in any of Claims 1 to 16 and another substance which is compatible therewith.

18. A composition as claimed in Claim 17, wherein the other compatible substance is a fuel, an alcohol or a combustible fluid.

19. A process for the preparation of a compound as claimed in Claim 1, which comprises reacting a compound of general Formula I, (in which $R_f$, n, OA, m and n have the meanings defined in Claim 1, and at least some of the groups x are hydroxyl the remainder, if any, being nitrate groups) with nitric acid.

20. A method of improving the combustion of fuels, propellants or explosives, which comprises admixing therewith from 0.01 to 50% by weight of a compound as claimed in any one of Claims 1 to 16, and combusting and/or detonating the mixture.

21. A method of running an engine which comprises injecting into the engine, either separately or in combination, a nitrato compound as claimed in any of Claims 1 to 16 and a combustible fuel.

22. A fuel, for compression ignition engines, said fuel comprising from 0.01 to 10% by weight of a compound of any of Claims 1 to 16 in admixture with a combustible fluid.

23. A fuel as claimed in Claim 22, wherein the combustible fluid comprises one or more of methanol, ethanol, a $C_3$ to $C_{20}$ alcohol or a low cetane hydrocarbon.

0080314

European Patent Office

# EUROPEAN SEARCH REPORT

Application number

EP 82 30 6082

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl. 3) |
| X | FR-A-1 127 647 (ETAT FRANCAIS)<br>*The whole document* | 1,6-8, 19 | C 07 C 77/02<br>C 07 C 76/04<br>C 07 H 15/08<br>C 10 L 1/22<br>C 08 G 65/32 |
| X | US-A-2 378 466 (G.O.CURME Jr.)<br>*The whole document* | 1-3,17-23 | |
| | | | TECHNICAL FIELDS SEARCHED (Int. Cl. 3) |
| | | | C 07 C 76/00<br>C 07 C 77/00<br>C 07 H 15/00<br>C 10 L 1/00<br>C 08 G 65/00 |

The present search report has been drawn up for all claims

| Place of search | Date of completion of the search | Examiner |
|---|---|---|
| THE HAGUE | 18-02-1983 | WRIGHT M.W. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82